(19) Europäisches Patentamt
European Patent Office
Office européen des brevets

(11) Publication number: **0 024 189**
**B1**

(12) **EUROPEAN PATENT SPECIFICATION**

(45) Date of publication of patent specification: **02.03.83**

(21) Application number: **80302769.7**

(22) Date of filing: **12.08.80**

(51) Int. Cl.$^3$: **C 07 H 19/00,**
C 07 D 493/06,
A 61 K 31/35, A 23 K 1/17,
A 61 K 31/70

(54) **Acylated laidlomycin derivatives, their preparation and compositions.**

(30) Priority: **13.08.79 US 65812**

(43) Date of publication of application:
**25.02.81 Bulletin 81/8**

(45) Publication of the grant of the patent:
**02.03.83 Bulletin 83/9**

(84) Designated Contracting States:
**AT BE CH DE FR GB IT LI LU NL SE**

(56) References cited:
**US - A - 3 839 557**

**JOURNAL OF ANTIBIOTICS, vol. XXIX, no. 7, page 759 F. KITAME: "The constitution of laidlomycin, a new antimoycoplasma antibiotic"**

(73) Proprietor: **SYNTEX (U.S.A.) INC.**
**3401 Hillview Avenue**
**Palo Alto, California 94304 (US)**

(72) Inventor: **Kluge, Arthur F.**
**1683 Parkhills Avenue**
**Los Altos California 94022 (US)**
Inventor: **Clark, Robin D.**
**2165 Bowdoin Street**
**Palo Alto California 94036 (US)**

(74) Representative: **Armitage, Ian Michael et al,**
**MEWBURN ELLIS & CO. 2/3 Cursitor Street**
**London EC4A 1BQ (GB)**

**EP 0 024 189 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Acylated laidlomycin derivatives, their preparation and compositions

This invention relates to novel esters of laidlomycin which are used to increase the feed efficiency in ruminants and to treat coccidiosis in domestic animals, especially in chickens.

The antibiotic laidlomycin is a known compound which inhibits the growth of some Gram-positive bacteria. See *The Journal of Antibiotics,* Vol. XXIX, No. 7, pp. 759—761, July, 1976 and Vol. XXVII, No. 11, pp. 884—888, Nov. 1974. Monensin is also a known antibiotic and has been shown to be useful in increasing feed utilization in ruminants. See for example, U.S. 3,794,732. The structure of each of these compounds is shown in Formula (I), below, wherein for laidlomycin R is propionate and A is hydrogen, for monensin (Factor A) R is methoxy and A is methyl, and for monensin (Factor B) R is methoxy and A is hydrogen.

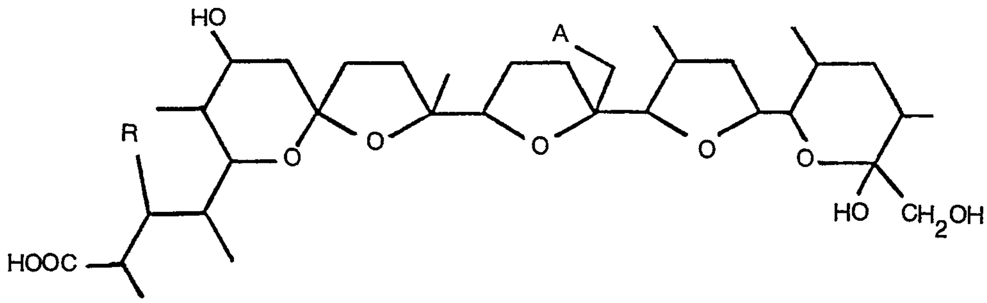

(I)

As discussed in the above-mentioned U.S. Patent 3,794,732, it is important to increase the efficiency of feed utilization in domestic animals, especially meat producing and milk producing animals such as cattle. Carbohydrates form a large part of these animals' diets, and the efficiency of carbohydrate utilization is desirably increased by treatments which encourage intraruminal production of propionate rather than acetate from the carbohydrates. The theory behind this is discussed in the '732 patent and is incorporated herein by reference.

The improved efficiency of feed utilization resulting from the use of compounds described by Formula (I), above, can be determined by observing an increased concentration or molar percentage of propionate in the rumen.

U.S. 3839557 discloses the use of certain antibiotics such as monensin or derivatives thereof, including the physiologically acceptable esters, for increasing feed utilisation in ruminants. No special advantages are ascribed to the esters.

Surprisingly, it has now been discovered that the laidlomycin esters of this invention exhibit an unexpectedly superior increase in the molar percentage of propionate produced by rumen microorganisms as compared to laidlomycin or the sodium salt thereof. It has also been found that the compounds of this invention inhibit lactic acid production better than laidlomycin or its sodium salt.

One aspect of this invention is a compound chosen from those represented by the formula

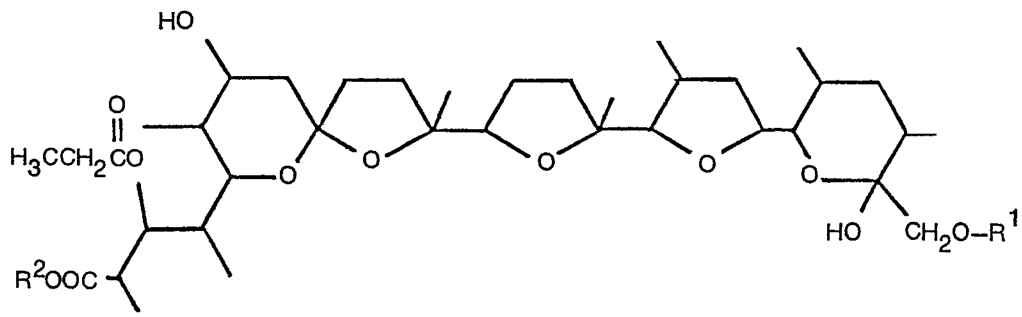

(A)

wherein $R^1$ is an aliphatic or alicyclic hydrocarbon acyl group of 2 through 18 carbon atoms and $R^2$ is hydrogen or an alkali metal cation (e.g. sodium, potassium or lithium).

Another aspect of the invention is the combination of a compound chosen from those represented by Formula (A), above, wherein $R^1$ and $R^2$ are as defined, with a suitable feed carrier for a ruminant animal.

A further aspect of this invention is a process for increasing the efficiency of feed utilization of an animal having a developed rumen function (a ruminant) which comprises orally administering to such animal a feed efficiency increasing amount of at least one compound represented by Formula (A) above, wherein $R^1$ and $R^2$ are as defined hereinabove.

Still another aspect of this invention is a compound of Formula (A) for use in controlling coccidial

infections in a warm blooded animal host having need for such treatment.

Still another aspect of this invention is a veterinary composition which comprises at least one compound represented by Formula (A) above (where $R^1$ and $R^2$ are as defined) and a veterinary pharmaceutically acceptable excipient.

Still another aspect of this invention is a process for preparing a compound represented by Formula (A) wherein $R^1$ and $R^2$ are as defined above, which process comprises reacting a compound of Formula (A) wherein $R^1$ is hydrogen and $R^2$ is as defined previously with a reactive derivative of an aliphatic hydrocarbon acid of two through eighteen carbons at temperatures of 0° to 30°C. Preferred reaction times are less than 10 hours.

The compounds of this invention include those compounds of Formula (A) wherein $R^2$ is hydrogen or an alkali metal salt and $R^1$ is an aliphatic or alicyclic hydrocarbon acyl group of two through eighteen carbon atoms. Each ester is derived from a reactive derivative of the corresponding acid containing 2 to 18 carbon atoms, i.e. straight chain, branched or cyclic aliphatic hydrocarbons. Thus, representative aliphatic $R^1O$— groups are, *inter alia*, acetate, propionate, butyrate, valerate, hexanoate, heptanoate, octanoate, nonanoate, decanoate, undecanoate, dodecanoate, tridecanoate, tetradecanoate, hexadecanoate, heptadecanoate, stearate or various branched alkanoates. Representative alicyclic $R^1O$— groups are cycloalkyl carboxylate moieties such as cyclopropylcarboxylate, cyclobutylcarboxylate, cyclopentylcarboxylate, cyclohexylcarboxylate, cycloheptylcarboxylate, cyclooctylcarboxylate or the like.

Of these esters, the alkanoates of 3—6 carbon atoms are preferred while alkanoates of 3 or 4 carbon atoms are particularly preferred, the butyrate being the most preferred compound.

The monoester compounds of the invention are readily prepared by reacting laidlomycin or an alkali metal salt thereof at low temperatures such as about 0—30°C with a suitable derivative of an alkanoic acid such as a suitable acid halide, e.g. an acid chloride, or an anhydride, for example

acetyl chloride,
propionyl chloride,
butyryl chloride,
valeryl chloride,
hexanoyl chloride,
heptanoyl chloride,
octanoyl chloride,
nonanoyl chloride,
decanoyl chloride,
undecanoyl chloride,
dodecanoyl chloride,
stearyl chloride,
cyclopropylcarboxylic chloride,
cyclobutylcarboxylic chloride,
cyclopentylcarboxylic chloride,
cyclohexylcarboxylic chloride,
the anhydrides corresponding to the above acid chlorides, (particularly the symmetric acid anhydrides) and the like.

The reaction is carried out in a suitable inert solvent, preferably a cyclic amine such as pyridine. Generally the reaction is finished in less than 10 hours, 3 hours being needed at 5°C for the formation of the butyrate. It is preferred to employ between 1 and 2 moles of acid halide per mol of laidlomycin, or to employ between 1 and 2 moles of acid anhydride per mole of laidlomycin.

Administration of a compound of this invention results in improved food utilization and also presents and treats ketosis, the abnormal increase of ketones in the body. The causative mechanism of ketosis is a deficient production of propionate compounds. A presently recommended treatment is administration of propionic acid or feeds which particularly produce propionate. Because the compounds of this invention encourage propionate production from ordinary feeds, feed efficiency is increased and the incidence of ketosis is reduced.

The compounds of this invention are most readily administered orally to the ruminants to be treated. The easiest way to administer the compounds is merely by mixing them with a suitable feed carrier and feeding the animals this animal feed.

However, the compounds can be usefully administered in other ways. For example, they can be combined with a suitable, non-toxic veterinary pharmaceutical excipient incorporated into tablets, drenches, boluses or capsules and dosed to the animals. Formulation of the compounds of this invention in such dosage forms can be accomplished by means or methods well known in the veterinary pharmaceutical art. Each individual dosage unit should contain a quantity of the feed efficiency improving compound which has a direct relation to the proper daily dose of the animals to be treated.

Capsules are readily produced by filling gelatin capsules with any desired form of the desired compound alone or diluted with a pharmaceutical excipient such as an inert powdered diluent, e.g. sugar, starch or purified crystalline cellulose in order to increase its volume for convenience in filling capsules.

Tablets of the compounds of this invention are made by conventional pharmaceutical processes. Manufacture of tablets is a well known and highly advanced art. In addition to the active ingredient, a tablet usually contains other pharmaceutical excipients such as a base, a disintegrator, an absorbant, a binder, and a lubricant. Typical bases include lactose, fine icing sugar, sodium chloride, starch and mannitol. Starch is also a good disintegrator as is alginic acid. Surface active agents such as sodium lauryl sulfate and dioctyl sodium sulfosuccinate are also used. Commonly used absorbants again include starch and lactose while magnesium carbonate is also useful for oily substances. Frequently used binders are gelatin, gums, starch, dextrin and various cellulose derivatives. Among the commonly used lubricants are magnesium stearate, paraffin wax, various metallic soaps and polyethylene glycol.

The method of the invention can also be practiced by the administration of the compound as a slow release bolus. Such boluses are made as tablets are made except that a means to delay the dissolution of the compound is provided. Boluses are made to release for lengthy periods. The slow dissolution is assisted by choosing a highly water-insoluble form of the compound. A substance such as iron filings is added to raise the density of the bolus and keep it static on the bottom of the rumen.

Dissolution of the compound is delayed by use of a matrix of insoluble materials in which the drug is embedded. For example, substances such vegetable waxes, purified mineral waxes, and water-insoluble polymeric materials are useful.

Drenches of the compounds are prepared most easily by choosing a water-soluble form of the compound. If an insoluble form is desired for some reason, a suspension may be made. Alternatively, a drench may be formulated as a solution in a physiologically acceptable solvent such as a polyethylene glycol.

Suspensions of insoluble forms of the compounds can be prepared in nonsolvents such as vegetable oils such as peanut, corn, or sesame oil; in a glycol such as propylene glycol or a polyethylene glycol; or in water, depending on the form of the compound chosen.

Suitable physiologically-acceptable adjuvants are necessary in order to keep the compound suspended. The adjuvants can be chosed from among the thickeners, such as carboxymethylcellulose, polyvinylpyrrolidone, gelatin and the alginates. Many classes of surfactants serve to suspend the compounds of this invention. For example, lecithin, alkylphenol polyethylene oxide adducts, naphthalenesulfonates, alkylbenzenesulfonates, and the polyoxyethylene sorbitan esters are useful for making suspensions in liquid non-solvents.

In addition many substances which effect the hydrophilicity, density, and surface tension of the liquid can assist in making suspension in individual cases. For example, silicone anti-foams, glycols, sorbitol, and sugars can be useful suspending agents.

The suspendable compounds may be offered to the grower as a suspension, or as a dry mixture of the compounds and adjuvants to be diluted before use.

These compounds may also be administered in the drinking water of the ruminants. Incorporation into drinking water is performed by adding a water-soluble or water-suspendable form of the desired antibiotic to the water in the proper amount. Formulation of the compound for addition to drinking water follows the same principles as formulation of drenches.

The most practical way to treat animals with the compounds of the invention is by the formulation of the compound into the feed supply. Any type of feed may be medicated with the compounds, including common dry feeds, liquid feeds, and pelleted feeds.

The methods of formulating drugs into animal feeds are well known. It is usual to make a concentrated drug premix as a raw material for medicated feeds. For example, typical drug premixes may contain from about 1 to about 400 grams of drug per pound of premix. The wide range results from the wide range of concentration of drug which may be desired in the final feed. Premixes may be either liquid or solid.

Generally the daily dosage of the compounds of this invention will be about 0.1 to 1.0 mg per kg body weight, preferably about 0.3 to 0.8 mg/kg. Thus, a 800 pound (about 360 kg) steer would receive about 36 to 360 mg and preferably about 109 to 290 mg.

The formulation of ruminant feeds containing the proper amount of the compounds for useful treatment is mainly a matter of arithmetic. It is necessary only to calculate the amount of compound which it is desired to administer to each animal, to take into account the amount of feed per day which the animal eats and the concentration of compound in the pre-mix to be used, and calculate the proper concentration of compound in the feed.

All of the methods of formulating, mixing, and pelleting foods which are normally used in the ruminant feed art are entirely appropriate for manufacturing feeds containing the compounds of the invention.

The compounds of this invention are also effective in controlling coccidial infection in warm blooded animal hosts. The animals in which these compounds are useful include ruminants such as cattle, sheep, and goats as well as a non-ruminants such as horses, pigs, chickens and particularly young chicks. The means of administration is similar to that for the treatment of ruminants and preferably is given orally, particularly to young chicks in their feed. The dosages useful for treating coccidial infections are from 0.1 to 3.0 mg/kg.

The following examples set forth methods for preparing representative compounds of this

invention and biological data for representative compounds of this invention. It is understood, of course, that the examples are not to be read as limiting the scope of the claimed subject matter, but are given by way of illustration of the preparation and use of the invention.

Example 1

A. Crude laidlomycin (4.8 grams) is chromatographed on silica gel using ethylacetate as the eluant. Eluting with 1.0 liter of ethyl acetate and collecting the 400—700 ml fractions affords a solution of sodium laidloymycin in ethylacetate. Removing the ethyl acetate under vacuum affords sodium laidlomycin (2.2 grams) having a melting point of 259—261 °C, with an Rf of 0.5 using ethyl acetate.

A solution of 150 milligrams of sodium laidlomycin in 5 mls of pyridine is cooled to 5°C and 100 milligrams of butyryl chloride is added. After three hours at 5°C, the mixture is taken into 25 mls of methylene chloride, washed with 25 mls of hydrochloric acid and 25 mls of brine, then dried over sodium sulfate. The methylene chloride solvent is removed under vacuum and the residue is chromatographed on silica gel using ethyl acetate. Eluting with 0.1 liter of ethyl acetate and collecting the 50—100 ml fractions gives 130 mgs of the monobutyrate of laidlomycin (Formula (A) where $R^1$ is $C(O)CH_2CH_2CH_3$ and $R^2$ is H), m.p. 77—80°C and further characterized by the Carbon-13 Magnetic Resonance (CMR) Data set forth in Table A.

B. By following in principle the above procedure but substituting other appropriate acid chlorides such as acetyl chloride, propionyl chloride, isobutyryl chloride, pentanoyl chloride, hexanoyl chloride, heptanoyl chloride, octanoyl chloride, decanoyl chloride, dodecanoyl chloride, stearyl chloride, cyclopropylcarbonyloxy chloride, cyclobutylcarbonyloxy chloride, cyclopentylcarbonyloxy chloride or cyclohexylcarbonyloxy chloride for butyryl chloride, the monoesters of laidlomycin (Formula (A) wherein $R^2$ is hydrogen and $R^1$ is an acyl group) are prepared as set forth below. In the following list, the parenthetical expression is the abbreviation for the compound shown, which abbreviation is later used in Table A to represent the compound

laidlomycin acetate ($L.C_2$), m.p. 115°C;
laidlomycin propionate ($L.C_3$), m.p. 65—67°C;
laidlomycin isobutyrate ($L.isoC_4$), m.p. 62—65°C;
laidlomycin pentanoate ($L.C_5$), m.p. 90—93°C;
laidlomycin hexanoate ($L.C_6$), m.p. 70—73°C;
laidlomycin heptanoate (oil), ir 1730 $cm^{-1}$;
laidlomycin octanoate (oil), ir 1730 $cm^{-1}$;
laidlomycin decanoate, m.p. 54—56°C;
laidlomycin dodecanoate (oil), ir 1730 $cm^{-1}$;
laidlomycin stearate ($L.C_{18}$); m.p. 43—45°C
laidlomycin cyclopropylcarboxylate (L.CPC), m.p. 85—87°C;
laidlomycin cyclobutylcarboxylate (L.CBC), m.p. 68—70°C;
laidlomycin cyclopentylcarboxylate, m.p. 32—34°C;
laidlomycin cyclohexylcarboxylate; and the like.

Selected compounds are further characterized with CMR Data in Table A. In numbering the carbons in the laidlomycin molecule, and esters thereof, the following convention was employed:

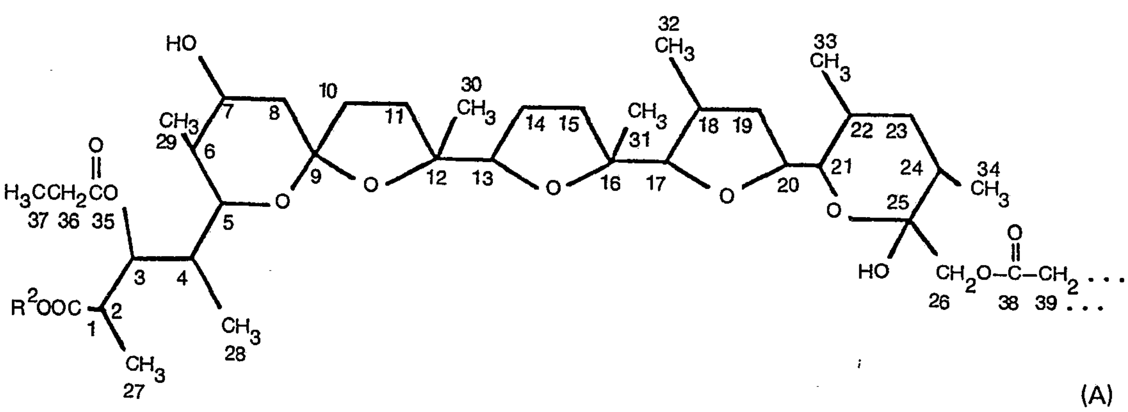

(A)

Carbons C—1, C—7, C—26, C—35, C—38 and C—39 were chosen as best distinguishing the compounds of this invention from the parent. In the column under C—39 . . . , the numbers are given which correspond to the acetate (C—39), the propionate (C—39, C—40), the butyrate (C—39, C—40), C—41), etc. Only the clearly identified peaks are chosen. Thus, in identifying the stearate only six peaks are set forth even though the stearate has 18 carbons.

TABLE A

CMR DATA
(PPM from tetramethylsilane)[1]

| COMPOUND | C—1 | C—7 | C—26 | C—35 | C—38 | C—39... |
|---|---|---|---|---|---|---|
| NaL. | 180.26 | 70.64 | 64.92 | 173.83 | — | — |
| $L.C_2$ | 180.85 | 70.45 | 64.34 | 173.86 | 171.03 | 21.03 |
| $L.C_3$ | 180.85 | 70.61 | 63.84 | 173.93 | 174.41 | 9.01, 27.56 |
| $L.C_4$ | 180.72 | 70.61 | 63.78 | 173.76 | 173.47 | 13.84, 18.21, 36.15 |
| L.CPC | 180.75 | 70.51 | 63.52 | 173.89 | 174.93 | 7.77, 8.42, 12.81 |
| $L.C_5$ | 180.85 | 70.51 | 63.81 | 173.92 | 173.92 | 13.82, 22.33, 27.96, 33.97 |
| L.CBC | 180.78 | 70.54 | 63.42 | 173.89 | 175.29 | 18.50, 24.67, 25.65, 38.17 |
| $L.C_6$ | 180.85 | 70.51 | 63.85 | 173.70 | 173.86 | 13.91, 22.43, 24.51, 31.37, 34.26 |
| $L.C_{18}$ | 180.82 | 70.51 | 63.78 | 173.73 | 173.89 | 14.14, 22.72, 24.58, 29.49, 29.71, 33.71 |

[1] Solvent: $CDCl_3$ treated with $K_2CO_3$—$D_2O$ using a Bruker 90 instrument.

Example 2 — Biological Activity

Enhancement of intraruminal propionic acid production is determined using a batch culture fermentation system. Rumen contents from a rumen-fistulated bovine are mixed with an equal amount of buffer which contains mineral, energy and nitrogen sources required for continued growth and metabolism of the mixed microbial population. Aliquots of this inoculum then are transferred to incubation vessels containing appropriate amounts of sodium laidlomycin (Na L.) (see Example 1 A), and L. butyrate ($L.C_4$) (prepared according to the procedure of Example 1A). After 9 hours of incubation at 39°C, concentrations of individual volatile fatty acids in each incubation vessel are determined by gas-liquid chromatography. The net production of each individual volatile fatty acid (acetic [AA], propionic [PA] and butyric [BA]) is calculated as the difference between the concentration after incubation and the concentration before incubation. The molar percentage of propionic acid produced is calculated from the following equation:

$$\%\ \text{propionic acid} = \frac{PA}{AA + PA + BA} \times 100$$

Inhibition of lactic acid accumulation is determined using a second batch culture fermentation system. Rumen contents from a rumen fistulated bovine are mixed with an equal amount of buffer which contains a 150 mM concentration of glucose. Following 6 hours of incubation at 39°C, lactic acid produced in the fermentation is analyzed by a specific enzymatic assay. The concentration of lactic acid in the fermentation broth is quantified by the increase in absorbance of light at 340 mm when nicotinamide adenine dinucleotide is reduced by lactic acid with the aid of lactate dehydrogenase.

The comparative activities of sodium laidlomycin (Na L.) and the laidlomycin butyrate ($L.C_4$) upon propionic acid and lactic acid production are set forth in Tables 1 and 2 where the concentration is in micrograms per ml (μg/ml).

TABLE 1

| Concentration of Test Compound (μg/ml) | % Propionic Acid | |
|---|---|---|
| | Na L. | L. $C_4$ |
| 10 | 45.4 | 53.9 |
| 5 | 44.4 | 49.3 |
| 2.5 | 43.2 | 52.2 |
| 1.25 | 41.3 | 46.1 |
| .62 | 40.8 | 46.4 |
| .31 | 40.2 | 43.6 |
| 0 | 35.4 | 35.4 |

TABLE 2

| Concentration of Test Compound (μg/ml) | Lactic Acid Accumulation (% of Control) | |
|---|---|---|
| | Na L. | L. $C_4$ |
| 10 | 67 | 13 |
| 5 | 93 | 13 |
| 2.5 | 112 | 11 |
| 1.25 | 125 | 8 |
| .62 | 132 | 30 |
| .31 | 135 | 71 |
| 0 | 100 | 100 |

From the results of these tests it is clear that the butyrate of laidlomycin demonstrates a surprisingly increased potency toward both enhancement of propionic acid production and inhibition of lactic acid accumulation.

Example 3 — Biological Activity

The procedure of Example 2 was followed to compare laidlomycin (L.), sodium L. (Na L.), L.-propionate ($L.C_3$), L. butyrate ($L.C_4$), L. isobutyrate (L. $isoC_4$), L. pentanoate ($L.C_5$) and L. hexanoate ($L.C_6$). The results are set forth in Tables 2 and 3.

TABLE 3

| Concentration of Test Compound ($\mu g/ml$) | % Propionic Acid | | | | | | |
|---|---|---|---|---|---|---|---|
| | L. | Na L. | L. $C_4$ | L. iso $C_4$ | L. $C_5$ | L. $C_6$ | L. $C_3$ |
| 20 | 43.2 | 36.8 | 46.9 | 47.5 | 39.7 | 41.7 | 44.4 |
| 5 | 39.8 | 34.6 | 43.7 | 43.9 | 36.1 | 40.1 | 41.9 |
| 1.25 | 36.0 | 34.1 | 39.9 | 39.6 | 33.9 | 37.1 | 37.8 |
| .31 | 32.1 | 32.0 | 35.5 | 34.3 | 30.6 | 33.0 | 32.9 |
| 0 | 28.5 | 28.5 | 28.5 | 28.5 | 28.5 | 28.5 | 28.5 |

TABLE 4

| Concentration of Test Compound ($\mu g/ml$) | Accumulation of Lactic Acid — % of Control | | | | | | |
|---|---|---|---|---|---|---|---|
| | L. | Na L. | L. $C_4$ | L. iso $C_4$ | L. $C_5$ | L. $C_6$ | L. $C_3$ |
| 20 | 16 | 40 | 19 | 16 | 11 | 14 | 14 |
| 5 | 20 | 57 | 15 | 13 | 13 | 11 | 13 |
| 1.25 | 83 | 94 | 13 | 12 | 54 | 18 | 14 |
| .31 | 121 | 114 | 38 | 37 | 109 | 60 | 71 |
| 0 | 100 | 100 | 100 | 100 | 100 | 100 | 100 |

Example 4

The procedure of Example 2 was followed to compare the activities of L. heptanoate (L. $C_7$), L. octanoate (L. $C_8$), L. decanoate (L. $C_{10}$) and L. dodecanoate (L. $C_{12}$) in increasing propionic acid production and inhibiting lactic acid production. The results are set forth in Tables 5 and 6.

TABLE 5

| Concentration of Test Compound ($\mu g/ml$) | % Propionic Acid | | | |
|---|---|---|---|---|
| | L. $C_7$ | L. $C_8$ | L. $C_{10}$ | L. $C_{12}$ |
| 20 | 47.2 | 49.4 | 48.1 | 48.0 |
| 10 | 48.1 | 47.5 | 46.6 | 47.7 |
| 5 | 48.4 | 48.2 | 46.7 | 46.8 |
| 2.5 | 46.9 | 47.3 | 47.1 | 46.1 |
| 1.25 | 46.2 | 46.4 | 45.5 | 45.3 |
| .63 | 45.3 | 43.9 | 43.6 | 43.8 |
| .31 | 41.4 | 42.7 | 39.9 | 42.0 |
| 0 | 35.5 | 35.5 | 35.5 | 35.5 |

TABLE 6

| Concentration of Test Compound (μg/ml) | Lactic Acid Accumulation (μ mol/ml) | | | |
|---|---|---|---|---|
| | L. $C_7$ | L. $C_8$ | L. $C_{10}$ | L. $C_{12}$ |
| 20 | 1.94 | 2.14 | 0.96 | 1.65 |
| 10 | 1.46 | 1.39 | 0.94 | 2.16 |
| 5 | 1.16 | 1.27 | 0.94 | 3.32 |
| 2.5 | 1.32 | 1.31 | 1.20 | 1.04 |
| 1.25 | 1.90 | 1.21 | 0.84 | 11.19 |
| .63 | 14.12 | 9.41 | 30.78 | 38.01 |
| .31 | 38.37 | 41.96 | 36.86 | 34.90 |
| 0 | 36.65 | 36.65 | 36.65 | 36.65 |

Example 5

The procedure of Example 2 was followed to compare the activities of laidlomycin (L.) acetate (L. $C_2$), L. pentanoate (L. $C_5$), L. stearate (L. $C_{18}$), L. cyclobutylcarboxylate, L. cyclopentylcarboxylate, L. cyclohexylcarboxylate and L. cyclopropylcarboxylate. The results are set forth in Tables 7 and 8.

TABLE 7

| Concentration of Test Compound (μg/ml) | % Propionic Acid | | | | | | |
|---|---|---|---|---|---|---|---|
| | L. $C_2$ | L. $C_5$ | L. $C_{18}$ | (Cyclo-butyl-carboxy-late) | (Cyclo-pentyl-carboxy-late) | (Cyclo-hexyl-carboxy-late) | (Cyclo-propyl-carboxy-late) |
| 20 | 53.0 | 51.1 | 45.8 | 51.2 | 50.0 | 53.3 | 50.7 |
| 10 | 51.2 | 51.6 | 45.5 | 51.8 | 51.6 | 51.8 | 49.6 |
| 5 | 52.2 | 49.9 | 44.6 | 51.7 | 51.1 | 51.6 | 50.9 |
| 2.5 | 50.4 | 50.9 | 41.6 | 52.5 | 50.3 | 50.8 | 49.3 |
| 1.25 | 50.7 | 48.7 | 40.0 | 50.0 | 51.9 | 50.1 | 49.7 |
| .625 | 47.1 | 48.1 | 37.6 | 47.1 | 47.9 | 49.0 | 46.4 |
| .313 | 42.8 | 45.9 | 37.1 | 47.5 | 47.1 | 47.2 | 47.7 |
| 0 | 36.1 | 36.1 | 36.1 | 36.1 | 36.1 | 36.1 | 36.1 |

TABLE 8

Lactic Acid Accumulation ($\mu$ mol/ml)

| Concentration of Test Compound ($\mu$g/ml) | L. $C_2$ | L. $C_5$ | L. $C_{18}$ | (Cyclo-butyl-carboxy-late) | (Cyclo-pentyl-carboxy-late) | (Cyclo-hexyl-carboxy-late) | (Cyclo-propyl-carboxy-late) |
|---|---|---|---|---|---|---|---|
| 20 | 5.28 | 5.13 | 29.93 | 4.75 | 9.66 | 5.72 | 3.96 |
| 10 | 4.35 | 4.63 | 37.83 | 4.73 | 4.53 | 4.97 | 3.53 |
| 5 | 4.35 | 4.04 | 46.20 | 4.32 | 4.08 | 4.83 | 3.17 |
| 2.5 | 4.34 | 3.75 | 52.51 | 4.01 | 3.73 | 4.40 | 3.16 |
| 1.25 | 6.89 | 4.67 | 48.87 | 3.82 | 3.63 | 3.74 | 2.50 |
| .625 | 22.23 | 11.91 | 52.57 | 8.05 | 6.79 | 12.78 | 2.12 |
| .313 | 41.19 | 24.62 | 50.29 | 23.25 | 24.75 | 35.55 | 15.54 |
| 0 | 40.47 | 40.47 | 40.47 | 40.47 | 40.47 | 40.47 | 40.47 |

Example 6

Five groups of male, Swiss-Webster mice (Simonsen), each mouse weighing about 25 grams, were dosed orally with 0.25 ml of a solution of laidlomycin butyrate in sesame oil. A control group of six mice received only sesame oil. The mice were observed daily for mortality for 21 days. The dosage and results are set forth in Table 9, below. From this information the $LD_{50}$ was calculated to be more than 200 mg/kg.

TABLE 9

| Group | No. Mice | Dosage (mg/kg) | No. Deaths/No. Mice |
|---|---|---|---|
| 1 | 6 | 0 | 0/6 |
| 2 | 6 | 12.5 | 0/6 |
| 3 | 6 | 25 | 0/6 |
| 4 | 6 | 50 | 0/6 |
| 5 | 6 | 100 | 0/6 |
| 6 | 6 | 200 | 0/6 |

Other compounds of this invention show similar toxicity characteristics.

Example 7

A premix formulation of the composition set forth in Table 10 is prepared as set forth below.

TABLE 10

| Component | % weight |
|---|---|
| Laidlomycin butyrate | 12.0 |
| Ethoxyquin | 0.01 |
| Rice Mill Hulls | 87.99 |

The ethoxyquin is mixed with 1.2% of the rice mill hulls to prepare an initial premix composition. This mixture is then thoroughly mixed with the remainder of the rice mill hulls whereupon the L. butyrate is added and thoroughly mixed to prepare the final formulation.

Example 8

Three hundred twenty (320) g of the sodium salt of laidlomycin is mixed with 6.4 l of methylene chloride ($CH_2Cl_2$) and 215 ml triethylamine (TEA) then cooled with stirring to −10°C under nitrogen. One hundred four (104) ml butyrylchloride (BuCl) is added over a twelve minute span as the temperature rises to −5°C. Over ten minute intervals six additions are made to the reaction vessel, with each addition being 15.4 ml TEA and subsequently 11.5 ml BuCl. The resulting reaction mixture is extracted twice with 2 l portions of aqueous $NaHCO_3$, once with a 2 l portion of water, twice with 2 l portions of 10% HCl, twice with 2 l portions of water and once with a 2 l portion of saturated brine. The organic phase is dried over $Na_2SO_4$, the solvent removed *in vacuo*, and the resulting oil is seeded, then stored overnight at −10°C. The resulting precipitate is slurried with 250 ml of 20% ether/hexane, 250 ml of hexane is added, and the mixture is cooled to −10°C for one hour then filtered to give 242.8 g of a product having a mp of 104—106°C (Product A). The solvents from the mother liquor are then concentrated to give another precipitate which is similarly treated with ether/hexane to give 73.4 g of a product having a mp of 93—96 (Product B). The solvents are removed from the mother liquor and 25 g $KHCO_3$ in 100 ml water, along with one l of methanol (MeOH) is added thereto. Four hundred ml of water is added to cause an oil to form. The oil is redissolved by adding 500 ml MeOH and the mixture is allowed to stand for two days; no precipitate forms. Two hundred (200) ml of water are added and the mixture is filtered to remove insolubles. Twenty-five (25) g of $KHCO_3$ is added to the filtrate and a precipitate forms within an hour. The mixture is stirred for two and a half days then filtered to give a product which, when dried, exhibits a mp of 133—136°C (Product C).

A mixture of 222.8 g of product A, 72.8 g of product B, and 29.1 g of product C is mixed with 1.5 l acetone, 75 g $KHCO_3$ and 150 ml water. Twenty-five (25) g of $KHCO_3$ is added after 30 minutes and another 25 g after another 30 minutes. The acetone is removed *in vacuo* and 500 ml MeOH added thereto. The resulting mixture is concentrated and 1.5 l of MeOH and 600 ml water is added thereto to give a precipitate. The mixture is transferred to another vessel, slurrying with extra aqueous MeOH and stirred for one hour. The precipitate is collected, washed 4 times with 250 ml of water, suctioned dry, and dried overnight in an oven to give 273.9 g of the potassium salt of laidlomycin butyrate (Formula (A)) where $R^1$ is $C(O)CH_2CH_2CH_3$ and $R^2$ is K), mp 143—148°C.

**Claims for the Contracting States: BE CH DE FR GB IT LI LU NL SE**

1. A compound chosen from those represented by the Formula

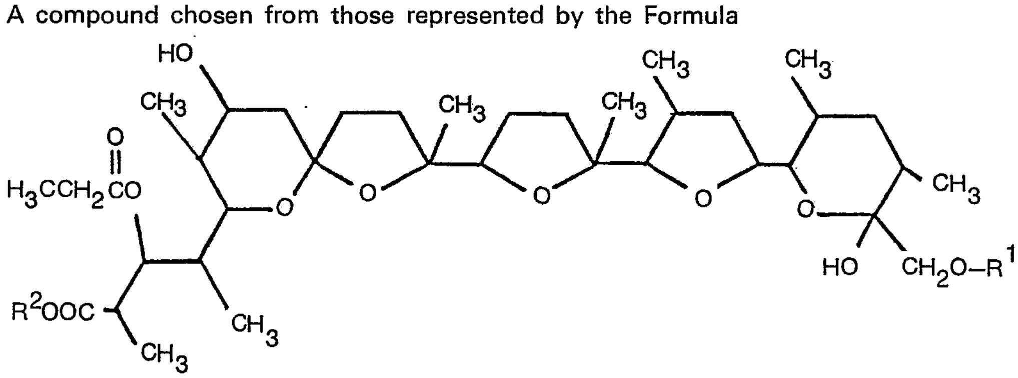

(A)

wherein $R^1$ is an aliphatic or alicyclic hydrocarbon acyl group of two to eighteen carbon atoms and $R^2$ is an alkali metal cation or hydrogen.

2. A compound of Claim 1 wherein $R^1$ is an aliphatic acyl of 3—6 carbon atoms and $R^2$ is hydrogen, sodium or potassium.

3. A compound of Claim 2 wherein $R^1$ is aliphatic acyl of 3 or 4 carbon atoms.

4. A compound of Claim 3 wherein $R^1$ is

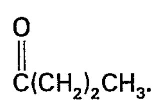

5. A method of increasing the efficiency of food utilization in a ruminant animal having a developed rumen function which comprises the oral administration to such animal of a propionate-increasing amount of a compound of any of claims 1 to 4.

6. A veterinary pharmaceutical composition which comprises a compound of any one of claims 1 to 4 in combination with a suitable feed carrier.

7. A composition for increasing the efficiency of feed utilization of a ruminant animal having a developed rumen function which comprises a compound of any one of claims 1 to 4 in combination with a pharmaceutically acceptable veterinary excipient.

8. An animal feed characterized by the content of a compound of any one of claims 1 to 4.

9. A process for preparing an acylated aliphatic or alicyclic laidlomycin derivative by reacting laidlomycin or an alkali metal salt thereof at 0 to 30°C with a corresponding aliphatic or alicyclic carboxylic acid halide or anhydride wherein said aliphatic or alicyclic acyl group has 2 to 18 carbon atoms, to form said laidlomycin derivative.

10. A compound of claim 1 wherein $R^1$ is selected from acetyl, butyryl, decanoyl, stearoyl, cyclopropylformyl and cyclopentylformyl.

11. A veterinary pharmaceutical composition containing a product compound of claim 9 or a compound of claim 10.

12. A feed composition containing a product compound of claim 9 or a compound of claim 10.

13. A compound of any one of claims 1 to 4 and 10 or a product compound of claim 9, for use in treating coccidiosis.

**Claims for the Contracting State: AT**

1. A method which comprises acylating laidlomycin to produce a compound of the Formula

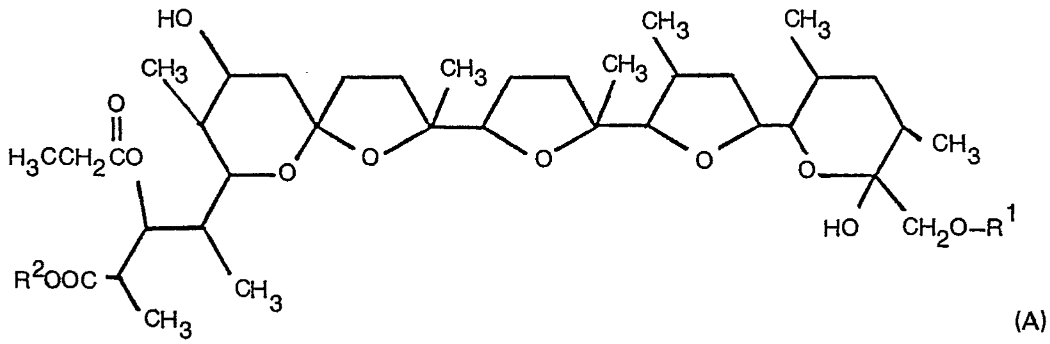

(A)

wherein $R^1$ is an aliphatic or alicyclic hydrocarbon acyl group of two to eighteen carbon atoms and $R^2$ is an alkali metal cation or hydrogen.

2. A method of claim 1 wherein $R^1$ is an aliphatic acyl of 3—6 carbon atoms and $R^2$ is hydrogen, sodium or potassium.

3. A method of claim 2 wherein $R^1$ is aliphatic acyl of 3 or 4 carbon atoms.

4. A method of claim 3 wherein $R^1$ is

$$\overset{O}{\overset{\|}{C}}(CH_2)_2CH_3.$$

5. A process for preparing an acylated aliphatic or alicyclic laidlomycin derivative by reacting laidlomycin or an alkali metal salt thereof at 0 to 30°C with a corresponding aliphatic or alicyclic carboxylic acid halide or anhydride wherein said aliphatic or alicyclic acyl group has 2 to 18 carbon atoms, to form said laidlomycin derivative.

6. A process according to claim 1 wherein $R^1$ is acetyl, butyryl, decanoyl, stearoyl, cyclopropylformyl or cyclopentylformyl.

7. A method of increasing the efficiency of food utilization in a ruminant animal having a developed rumen function which comprises the oral administration to such animal of a propionate-increasing amount of a compound of any one of claims 1 to 4.

8. For use in treating coccidiosis: a compound of the formula

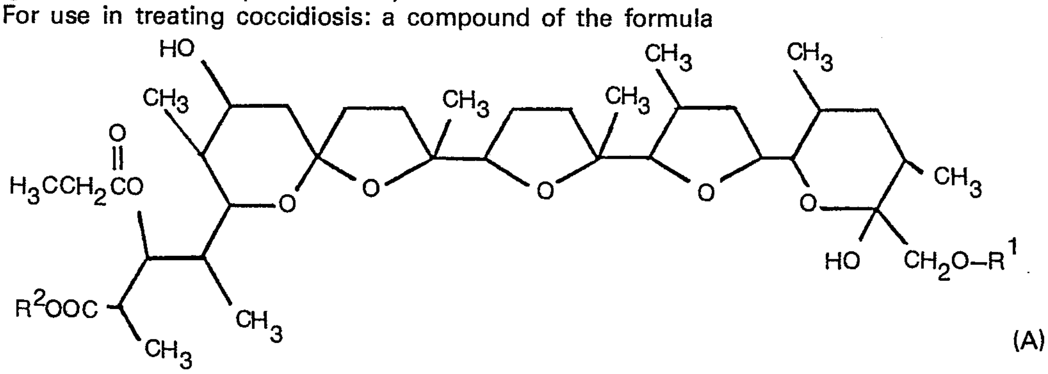

(A)

wherein $R^1$ is an aliphatic or alicyclic hydrocarbon acyl group of two to eighteen carbon atoms and $R^2$ is an alkali metal cation of hydrogen.

9. For use in the treatment of coccidiosis in warm-blooded non-human animals: a compound of the formula

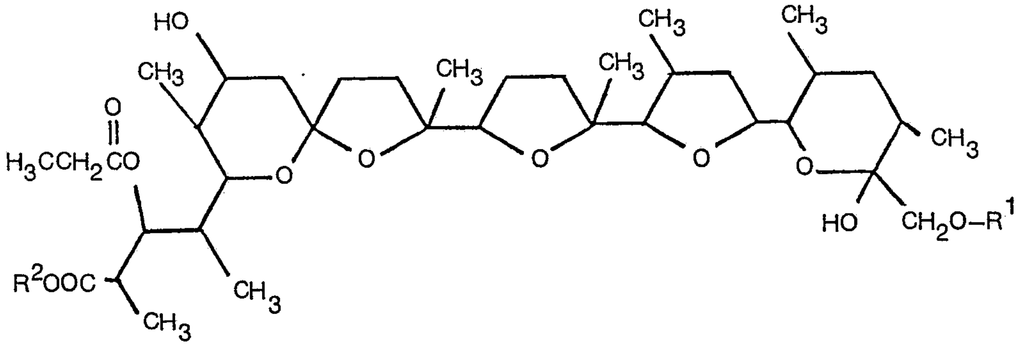

(A)

wherein $R^1$ is an aliphatic or alicyclic hydrocarbon acyl group of two to eighteen carbon atoms and $R^2$ is an alkali metal cation or hydrogen.

**Patentansprüche für die Vertragsstaaten: BE CH DE FR GB IT LI LU NL SE**

1. Eine Verbindung, ausgewählt unter den durch die Formel

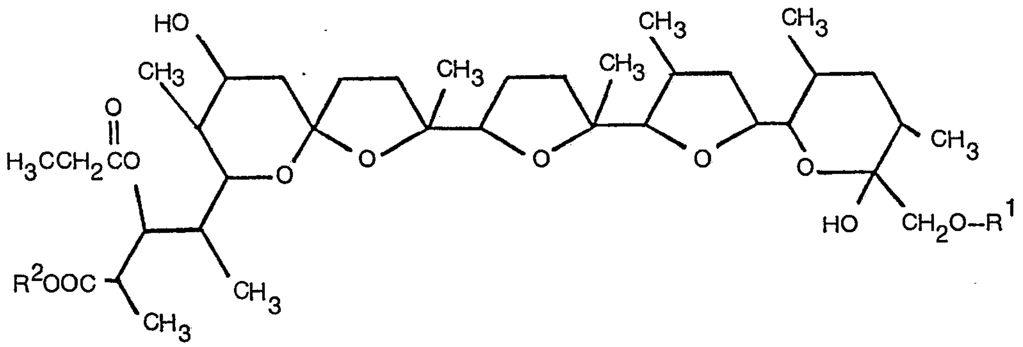

(A)

dargestellten Verbindungen, worin $R^1$ eine aliphatische oder alicyclische Kohlenwasserstoffacylgruppe mit 2 bis 18 Kohlenstoffatomen bedeutet und $R^2$ ein Alkalimetallkation oder Wasserstoff darstellt.

2. Eine Verbindung nach Anspruch 1, worin $R^1$ ein aliphatisches Acyl mit 3 bis 6 Kohlenstoffatomen bedeutet und $R^2$ Wasserstoff, Natrium oder Kalium darstellt.

3. Eine Verbindung nach Anspruch 2, worin $R^2$ aliphatisches Acyl mit 3 oder 4 Kohlenstoffatomen bedeutet.

4. Eine Verbindung nach Anspruch 3, worin $R^1$ für

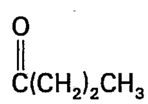

steht.

5. Ein Verfahren zur Steigerung der Effizienz der Futterausnützung bei einem wiederkäuenden Tier mit einer entwickelten Rumen-Funktion, welches Verfahren die orale Verabreichung einer Propionat-steigernden Menge einer Verbindung nach einem der Ansprüche 1 bis 4 an ein solches Tier umfaßt.

6. Eine veterinär-pharmazeutische Zusammensetzung, welche eine Verbindung nach einem der Ansprüche 1 bis 4 in Kombination mit einem geeigneten Futterträger umfaßt.

7. Eine Zusammensetzung zur Steigerung der Effizienz der Futterausnützung eines wiederkäuenden Tieres mit einer entwickelten Rumen-Funktion, welche eine Verbindung nach einem der Ansprüche 1 bis 4 in Kombination mit einem pharmazeutisch annehmbaren veterinären Excipiens umfaßt.

8. Ein Tierfutter, gekennzeichnet durch den Gehalt an einer Verbindung nach einem der Ansprüche 1 bis 4.

9. Ein Verfahren zur Herstellung eines acylierten aliphatischen oder alicyclischen Laidlomycin-Derivats durch Umsetzen von Laidlomycin oder eines Alkalimetallsalzes hievon bei 0 bis 30°C mit einem entsprechenden aliphatischen oder alicyclischen Carbonsäurehalogenid oder -anhydrid, worin

die genannte aliphatische oder alicyclische Acylgruppe 2 bis 18 Kohlenstoffatome umfaßt, zur Ausbildung des genannten Laidlomycin-Derivats.

10. Eine Verbindung nach Anspruch 1, worin $R^1$ ausgewählt ist aus Acetyl, Butyryl, Decanoyl, Stearoyl, Cyclopropylformyl und Cyclopentylformyl.

11. Eine verterinär-pharmazeutische Zusammensetzung, enthaltend eine Produktverbindung gemäß Anspruch 9 oder eine Verbindung gemäß Anspruch 10.

12. Eine Futter-Zusammensetzung, enthaltend eine Produktverbindung gemäß Anspruch 9 oder eine Verbindung gemäß Anspruch 10.

13. Eine Verbindung nach einem der Ansprüche 1 bis 4 und 10 oder eine Produktverbindung gemäß Beispiel 9, zur Anwendung in der Behandlung von Coccidiose.

**Patentansprüche für den Vertragsstaat: AT**

1. Ein Verfahren, das die Acylierung von Laidlomycin zur Bildung einer Verbindung der Formel

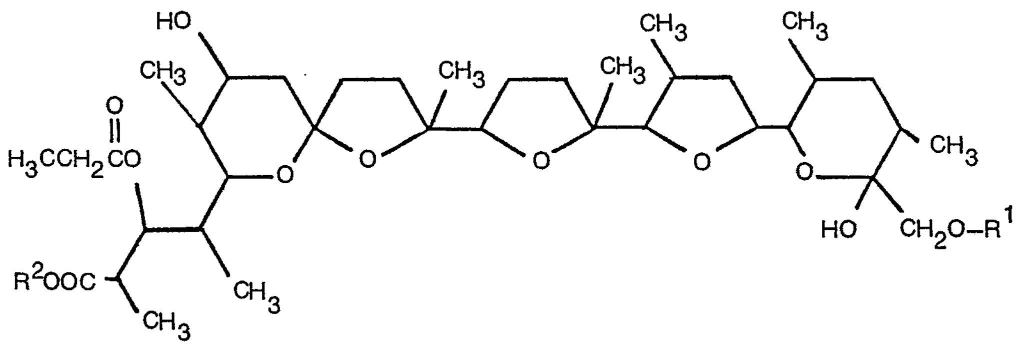

(A)

umfaßt, worin $R^1$ eine aliphatische oder alicyclische Kohlenwasserstoffacylgruppe mit 2 bis 18 Kohlenstoffatomen bedeutet und $R^2$ ein Alkalimetallkation oder Wasserstoff darstellt.

2. Ein Verfahren nach Anspruch 1, worin $R^1$ ein aliphatisches Acyl mit 3 bis 6 Kohlenstoffatomen und $R^2$ Wasserstoff, Natrium oder Kalium bedeuten.

3. Ein Verfahren nach Anspruch 2, worin $R^1$ aliphatisches Acyl mit 3 oder 4 Kohlenstoffatomen bedeutet.

4. Ein Verfahren nach Anspruch 3, worin $R^1$ für

$$\overset{\displaystyle O}{\overset{\|}{C}}(CH_2)_2CH_3$$

steht.

5. Ein Verfahren zur Herstellung eines acylierten aliphatischen oder alicyclischen Laidlomycinderivats durch Umsetzung von Laidlomycin oder eines Alkalimetallsalzes hievon bei 0 bis 30°C mit einem entsprechenden aliphatischen oder alicyclischen Carbonsäurehalogenid oder -anhydrid, worin die genannte aliphatische oder alicyclische Acylgruppe 2 bis 18 Kohlenstoffatome aufweist, zur Ausbildung des genannten Laidlomycinderivats.

6. Ein Verfahren nach Anspruch 1, worin $R^1$ Acetyl, Butyryl, Decanoyl, Stearoyl, Cyclopropylformyl oder Cyclopentylformyl bedeutet.

7. Ein Verfahren zur Steigerung der Effizienz der Futterausnützung bei einem wiederkäuenden Tier mit einer entwickelten Rumen-Funktion, welches die orale Verabreichung einer Propionat-steigernden Menge einer Verbindung nach einem der Ansprüche 1 bis 4 an ein solches Tier umfaßt.

8. Zur Anwendung bei der Behandlung von Coccidiose: eine Verbindung der Formel

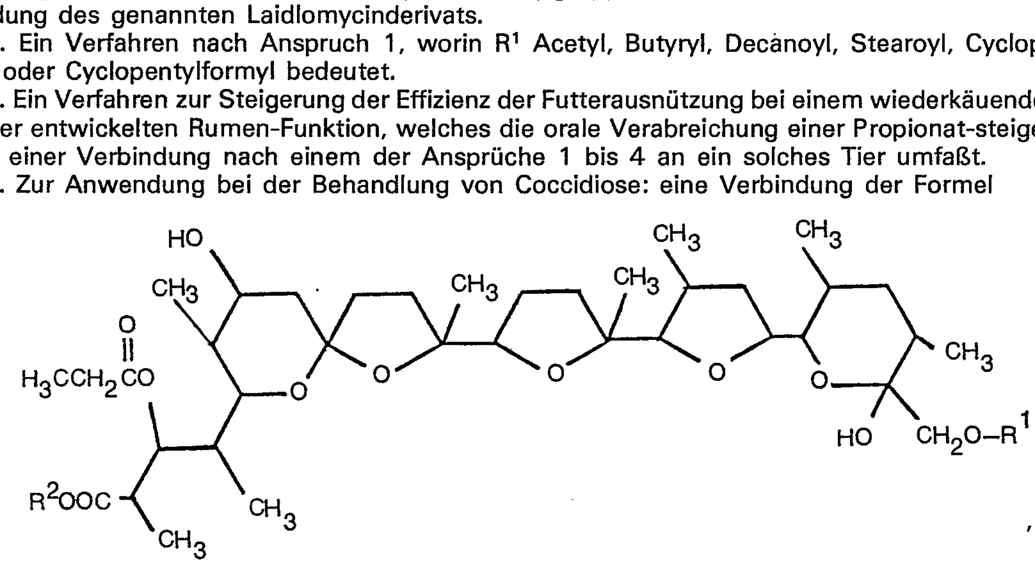

, (A)

worin $R^1$ eine aliphatische oder alicyclische Kohlenwasserstoffacylgruppe mit 2 bis 18 Kohlenstoffatomen bedeutet und $R^2$ ein Alkalimetallkation oder Wasserstoff darstellt.

9. Zur Anwendung in der Behandlung von Coccidose bei warmblütigen, vom Menschen verschiedenen Tieren: eine Verbindung der Formel

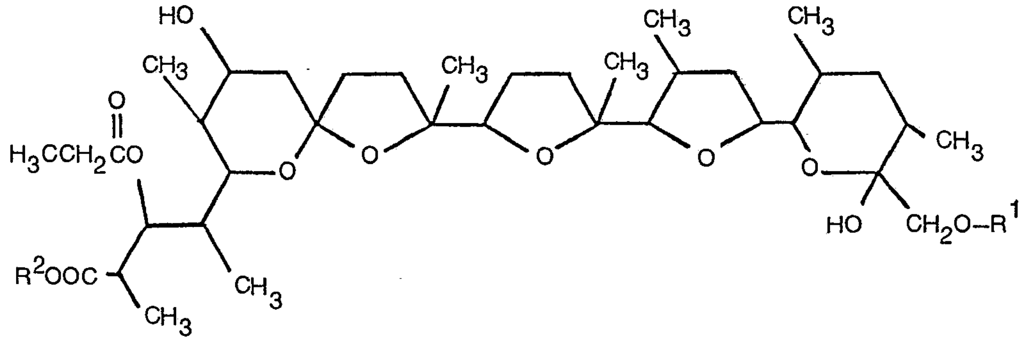

. (A)

worin $R^1$ eine aliphatische oder alicyclische Kohlenwasserstoffacylgruppe mit 2 bis 18 Kohlenstoffatomen bedeutet und $R^2$ ein Alkalimetallkation oder Wasserstoff darstellt.

**Revendications pour les Etats contractants: BE CH DE FR GB IT LI LU NL SE**

1. Composé choisi parmi ceux répondant à la formule:

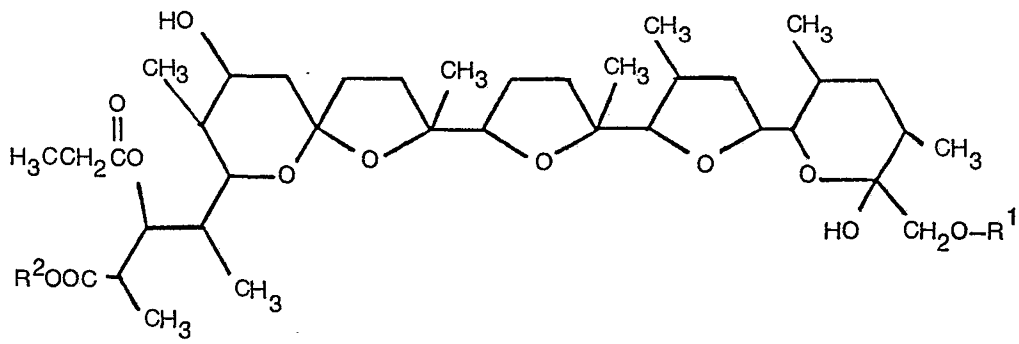

(A)

dans laquelle $R^1$ représente un groupe acyle d'hydrocarbure aliphatique ou alicyclique contenant 2 à 18 atomes de carbone et $R^2$ représente un atome d'hydrogène ou un cation d'un métal alcalin.

2. Composé suivant la revendication 1, caractérisé en ce que $R^1$ représente un groupe acyle aliphatique contenant 3 à 6 atomes de carbone et $R^2$ représente un atome d'hydrogène, de sodium ou de potassium.

3. Composé suivant la revendication 2, caractérisé en ce que $R^1$ représente un groupe aliphatique contenant 3 ou 4 atomes de carbone.

4. Composé suivant la revendication 3, caractérisé en ce que $R^1$ représente.

$$\overset{O}{\overset{\|}{C}}(CH_2)_2CH_3.$$

5. Procédé en vue d'accroître l'efficacité de l'utilisation des aliments chez un animal ruminant ayant une fonction développée de la panse, caractérisé en ce qu'il consiste à administrer par voie orale à cet animal, un composé suivant l'une quelconque des revendications 1 à 4 en une quantité accroissant la production de propionate.

6. Composition pharmaceutique vétérinaire, caractérisée en ce qu'elle comprend un composé suivant l'une quelconque des revendications 1 à 4, en combinaison avec un support approprié pour aliments.

7. Composition en vue d'accroître l'efficacité de l'utilisation des aliments chez un animal ruminant ayant une fonction développée de la panse, caractérisée en ce qu'elle comprend un composé suivant l'une quelconque des revendications 1 à 4 en combinaison avec un excipient vétérinaire pharmaceutiquement acceptable.

8. Aliment pour animaux, caractérisé en ce qu'il contient un composé suivant l'une quelconque des revendications 1 à 4.

9. Procédé de préparation d'un dérivé aliphatique ou alicyclique acylé de laidlomycine en faisant réagir de la laidlomycine ou un des ses sels de métaux alcalins à une température de 0 à 30°C avec un

anhydride ou un halogénure d'acide carboxylique alicyclique ou aliphatique correspondant dans lequel le groupe aryle alicyclique ou aliphatique contient 2 à 18 atomes de carbone, pour former ce dérivé de laidlomycine.

10. Composé suivant la revendication 1, caractérisé en ce que $R^1$ est choisi parmi les groupes acétyle, butyryle, décanoyle, stéaroyle, cyclopropylformyle et cyclopentylformyle.

11. Composition pharmaceutique vétérinaire contenant un composé obtenu par le procédé suivant la revendication 9 ou un composé suivant la revendication 10.

12. Composition alimentaire contenant un composé obtenu par le procédé suivant la revendication 9 ou un composé suivant la revendication 10.

13. Composé suivant l'une quelconque des revendications 1 à 4 et 10 ou composé obtenu par le procédé suivant la revendication 9 en vue de l'utiliser pour le traitement de la coccidiose.

**Revendications pour l'Etat contractant: AT**

1. Procédé consistant à acyler de la laidlomycine pour obtenir un composé de formule:

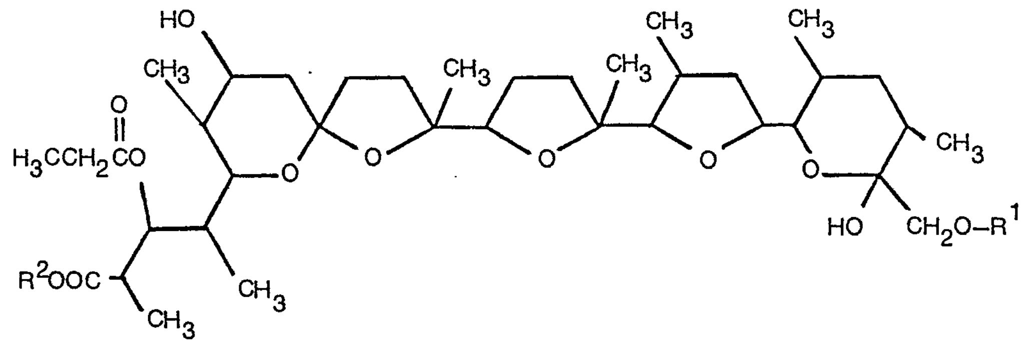

(A)

dans laquelle $R^1$ représente un groupe acyle d'hydrocarbure aliphatique ou alicyclique contenant 2 à 18 atomes de carbone et $R^2$ représente un atome d'hydrogène ou un cation d'un métal alcalin.

2. Procédé suivant la revendication 1, caractérisé en ce que $R^1$ représente un groupe acyle aliphatique contenant 3 à 6 atomes de carbone et $R^2$ représente un atome d'hydrogène, de sodium ou de potassium.

3. Procédé suivant la revendication 2, caractérisé en ce que $R^1$ représente un groupe acyle aliphatique contenant 3 ou 4 atomes de carbone.

4. Procédé suivant la revendication 3, caractérisé en ce que $R^1$ représente

$$\overset{O}{\overset{\|}{C}}(CH_2)_2CH_3.$$

5. Procédé de préparation d'un dérivé aliphatique ou alicyclique acylé de laidlomycine en faisant réagir de la laidlomycine ou un de ses sels de métaux alcalins à une température de 0 à 30°C avec un anhydride ou un halogénure d'acide carboxylique aliphatique ou alicyclique correspondant dans lequel le groupe aryle aliphatique ou alicyclique contient 2 à 18 atomes de carbone, pour former ce dérivé de laidlomycine.

6. Procédé suivant la revendication 1, caractérisé en ce que $R^1$ représente un groupe acétyle, butyryle, décanoyle, stéaroyle, cyclopropylformyle ou cyclopentylformyle.

7. Procédé en vue d'accroître l'efficacité de l'utilisation des aliments chez un animal ruminant ayant une fonction développée de la panse, caractérisé en ce qu'il consiste à administrer par voie orale à cet animal, un composé suivant l'une quelconque des revendications 1 à 4 en une quantité accroissant la production de propionate.

8. En vue de l'utiliser pour le traitement de la coccidiose: un composé de formule:

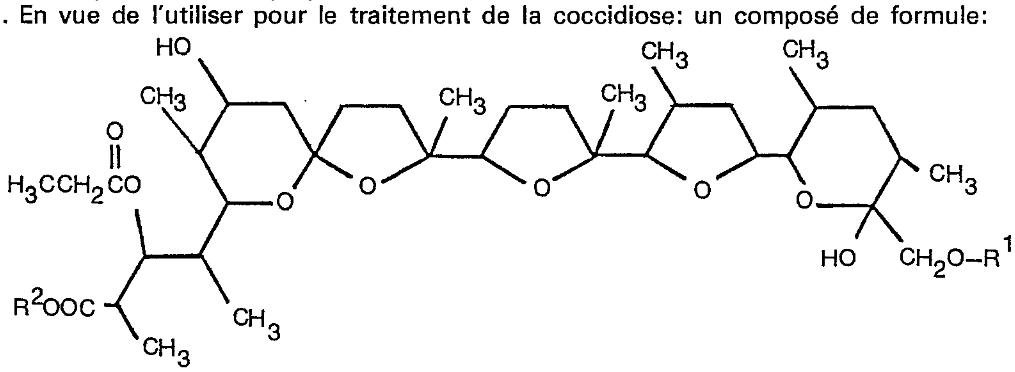

(A)

dans laquelle $R^1$ représente un groupe acyle d'hydrocarbure aliphatique ou alicyclique contenant 2 à 18 atomes de carbone et $R^2$ représente un atome d'hydrogène ou un cation d'un métal alcalin.

9. En vue de l'utiliser pour le traitement de la coccidiose chez des animaux à sang chaud, mais non chez l'homme: un composé de formule:

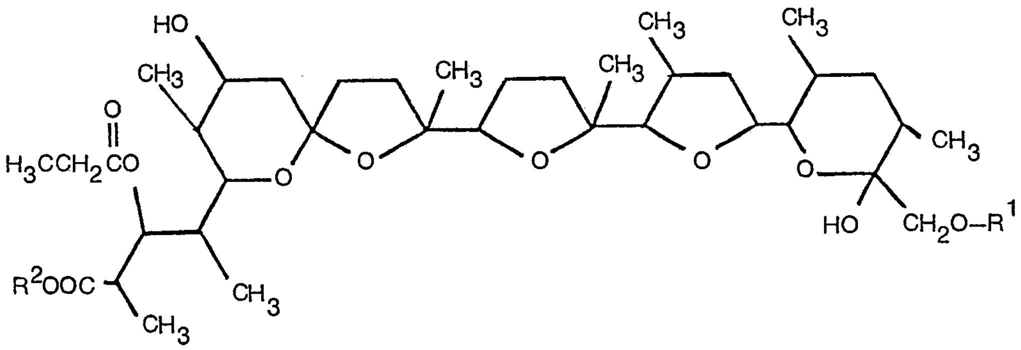

(A)

dans laquelle $R^1$ représente un groupe acyle d'hydrocarbure aliphatique ou alicyclique contenant 2 à 18 atomes de carbone et $R^2$ représente un atome d'hydrogène ou un cation d'un métal alcalin.